# DEMANDE DE BREVET EUROPEEN

(11) **EP 3 725 316 A1**
(43) Date de publication de la demande: **21.10.2020**
(21) Numéro de dépôt: 19315023.2
(22) Date de dépôt: 16.04.2019
(51) Int. Cl.: A61K 31/565, A61P 21/00

(54) **UTILISATION DU FULVESTRANT POUR LA PRISE EN CHARGE THERAPEUTIQUE DE LA MYOPATHIE A CORES**

(71) Demandeur: Université Grenoble Alpes, 38400 Saint-Martin-d'Hères (FR); Institut National de la Santé et de la Recherche Médicale, 75013 Paris (FR); Centre Hospitalier Universitaire Grenoble Alpes, 38700 La Tronche (FR); Association Française contre les Myopathies, 75651 Paris Cedex 13 (FR)
(72) Inventeur: MARTY, Isabelle, 38113 Veurey (FR); PELLETIER, Laurent, 38240 Meylan (FR); TRAVARD, Lauriane, 38690 Colombe (FR)
(74) Mandataire: Cabinet Chaillot

(57) **Abrégé**

L'invention concerne un antagoniste du récepteur des oestrogènes de formule : 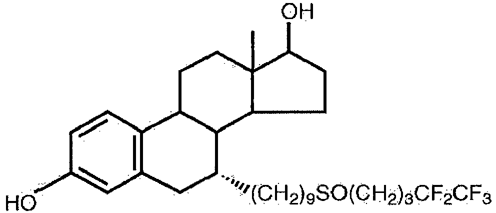 pour l'utilisation dans le traitement d'une maladie ou d'un trouble lié(e) à une baisse de la libération de calcium entre le réticulum sarcoplasmique et le cytosol, en particulier la maladie ou le trouble est une myopathie liée à une ou plusieurs mutations du gène RyR1 ou une myopathie liée à une baisse de la libération de calcium, plus particulièrement la myopathie à cores.

L'invention concerne également une composition pharmaceutique comprenant au moins l'antagoniste du récepteur des oestrogènes de l'invention et au moins un excipient pharmaceutiquement acceptable.

## Description

### ARRIERE-PLAN DE L'INVENTION

La présente invention concerne le domaine du traitement de pathologies liées à une baisse du relâchement de calcium, et porte en particulier sur l'utilisation du fulvestrant pour la prise en charge thérapeutique de la myopathie à cores.

La myopathie à cores est une maladie génétique caractérisée par un déficit de force musculaire et un retard du développement musculaire. Elle est principalement associée à des mutations dans le gène du récepteur de la ryanodine de type I (RyRl). RyRl est en réalité un canal calcique permettant la sortie du calcium du réticulum sarcoplasmique vers le cytosol. Dans le cytosol, le calcium induit le glissement des myofibrilles à l'origine de la contraction musculaire.

L'importance de RyRl dans la cascade d'événements menant de la stimulation de la fibre musculaire par le motoneurone à sa contraction explique parfaitement qu'une mutation du gène correspondant, altérant son expression ou sa fonction, induise un défaut de force musculaire.

En effet, la contraction du muscle squelettique comprend plusieurs étapes. Une stimulation provenant du motoneurone provoque d'abord la dépolarisation de la membrane plasmique de la fibre musculaire. Cette dépolarisation se propage jusqu'aux triades, des structures constituées d'une invagination de la membrane plasmique, le tubule T, associé à deux citernes terminales de reticulum sarcoplasmique. Là, elle active le récepteur des dihydropyridines (DHPR) situé dans la membrane du tubule T, qui active à son tour le récepteur de la ryanodine de type 1 (RyRl), auquel il est couplé mécaniquement. Ce second canal calcique situé dans la membrane du réticulum sarcoplasmique est responsable de la libération (ou relâchement) de calcium du réticulum vers le cytosol. Ce relâchement calcique hors des stocks intracellulaires permet enfin le glissement des filaments de myosine sur les filaments d'actine, et donc la contraction musculaire. Le calcium est ensuite repompé vers le réticulum.

A l'heure actuelle, il n'existe aucun traitement spécifique pour la prise en charge des patients atteints de la myopathie à cores. Les seuls soins apportés aux malades sont de la kinésithérapie et des corticoïdes pour améliorer l'état général, mais il n'existe en effet aucun traitement curatif.

Les recherches actuelles portent principalement sur la mise au point de thérapies géniques visant à corriger la mutation ou ses conséquences immédiates. Le problème réside principalement dans le fait qu'il existe de nombreuses mutations de RyR1 liées à la myopathie à cores, par conséquent, chaque stratégie de thérapie génique ne peut donc s'adresser qu'à un patient particulier ou un petit nombre de patients.

Des travaux publiés en 2013 (Dorchies et al., 2013) mettaient en évidence les propriétés thérapeutiques du tamoxifène chez des souris représentant un modèle de la dystrophie de Duchenne. Outre l'amélioration de la force de ces souris une fois traitées, les auteurs ont noté de nombreuses modifications histologiques et fonctionnelles sans toutefois être en mesure d'expliquer l'efficacité de cet agent. Ce même groupe de recherche a également abordé l'effet thérapeutique du tamoxifène sur une autre maladie musculaire, la myopathie myotubulaire.

La dystrophie de Duchenne et la myopathie myotubulaire ne sont pas étroitement apparentées, mais dans les deux cas, des altérations du relâchement du calcium ont été observées (De Luca et al., 2001, Plant et al., 2003, Dowling et al. 2009 et 2010). La piste mécanistique n'est pas abordée expérimentalement ni même évoquée dans la publication de Dorchies et al.

Les présents inventeurs ont alors développé une nouvelle stratégie thérapeutique pour leur pathologie d'intérêt, la myopathie à cores, partant du principe que le tamoxifène est un régulateur spécifique des récepteurs des oestrogènes. A ce titre, il module l'effet que les oestrogènes ont sur de nombreux tissus. Cependant, l'effet du tamoxifène est complexe car il exerce parfois un effet antagoniste sur les tissus (sein) et parfois agoniste (utérus, os). Son effet n'est pas caractérisé au niveau musculaire.

Pour surmonter l'incertitude de l'effet agoniste/antagoniste du tamoxifène sur le tissu musculaire, les inventeurs ont porté leur attention sur le fulvestrant, un antagoniste pur des récepteurs des oestrogènes.

Le fulvestrant est une molécule connue, à l'heure actuelle prescrite dans le cadre de la prise en charge des cancers du sein hormono-dépendants. Il possède dans ce cadre la même activité que le tamoxifène.

Dans la présente invention, la stratégie thérapeutique consiste en la recherche de l'amélioration du relâchement calcique du réticulum sarcoplasmique vers le cytosol qui est une étape déterminante pour la contraction musculaire. L'utilisation du fulvestrant dans la prise en charge thérapeutique de la myopathie à cores constitue la solution identifiée par les inventeurs pour pallier l'absence de traitement spécifique de cette myopathie.

### BREVE DESCRIPTION DE L'INVENTION

Les inventeurs disposent d'un modèle murin de la myopathie à cores qu'ils ont développé au laboratoire et caractérisé. Ce modèle est à la base d'une recherche de nouvelles stratégies thérapeutiques à large spectre, en opposition aux thérapies géniques basées sur la correction des mutations qui sont si nombreuses qu'elles sont quasiment spécifiques à chaque patient atteint de myopathie à cores.

L'intérêt de la stratégie thérapeutique de la présente invention permet de s'adresser à une large population de malades, l'agent devant cibler un mécanisme commun, indépendamment de la mutation responsable de la baisse de RyRl.

Les inventeurs ont relevé que la littérature fait mention du tamoxifène, un modulateur des récepteurs aux oestrogènes qui améliore les symptômes de deux pathologies musculaires, à savoir la dystrophie de Duchenne et la myopathie myotubulaire, sans pour autant que les auteurs aient pu élucider les mécanismes responsables de ces améliorations. Ces pathologies présentant quelques caractéristiques que partage la myopathie à cores, les inventeurs ont évalué ce traitement.

Les récepteurs des oestrogènes sont des protéines intracellulaires de la famille des récepteurs des stéroïdes qui possèdent donc une fonction de facteur de transcription. Il existe deux types de récepteurs des oestrogènes, ERα et ERβ, codés par deux gènes différents : *ESR1* et *ESR2,* respectivement. L'épissage alternatif des ARNm est à l'origine de plusieurs isoformes pour chaque type de récepteur. Leur profil d'expression dépend du tissu. L'expression relative variable des isoformes, celle des facteurs partenaires participant l'activation ou l'inhibition de la transcription, ainsi que l'existence d'effets indépendant des récepteurs, rendent la signalisation des oestrogènes particulièrement complexe (Kulkoyluoglu et al., 2016). L'action du tamoxifène est complexe : celui-ci agit comme agoniste ou antagoniste des récepteurs aux oestrogènes de façon tissu-dépendante (Berry et al., 1990), dépendant notamment des proportions dans lesquelles sont exprimés les deux récepteurs. Il a en effet une action agoniste à travers ERα, et antagoniste à travers ERβ (Salvatori et al., 2003).

L'action du tamoxifène étant complexe, les inventeurs ont décidé de porter leur attention sur un agent ayant une modalité d'action plus simple. Ils se sont donc concentrés sur un antagoniste pur des récepteurs aux oestrogènes ERα et ERβ, le fulvestrant. Ils ont alors administré le fulvestrant à des souris chez lesquelles ils ont induit une myopathie à cores et observé que malgré la perte de poids associée au développement de la maladie, les souris ne perdaient pas de force sur toute la durée du traitement (75 jours). Ils ont également effectué des études in vitro sur des cellules satellites murines primaires de muscles squelettiques, ainsi que sur des cellules humaines immortalisées issues d'une biopsie d'un patient atteint de myopathie à cores.

### DESCRIPTION DETAILLEE DE L'INVENTION

La présente invention concerne l'antagoniste du récepteur des oestrogènes de formule : 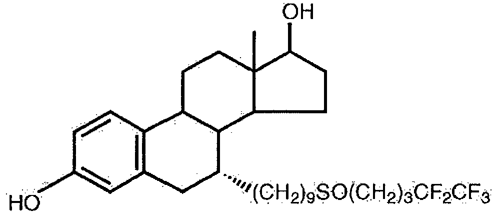 pour l'utilisation dans le traitement d'une maladie ou d'un trouble lié(e) à une baisse de la libération de calcium entre le réticulum sarcoplasmique et le cytosol.

Cet antagoniste est en particulier connu sous le nom de fulvestrant.

Dans un mode de réalisation, la maladie ou le trouble peut être une myopathie liée à une ou plusieurs mutations du gène RyR1 ou une myopathie liée à une baisse de la libération de calcium.

Dans un mode de réalisation particulier, la maladie ou le trouble peut être la myopathie à cores.

La myopathie à cores est une affection neuromusculaire caractérisée par des lésions rondes au centre de la fibre musculaire, visibles sur la biopsie musculaire, et par les manifestations cliniques d'une myopathie congénitale.

Selon un aspect, l'antagoniste est destiné à être administré à un sujet selon une quantité thérapeutiquement efficace.

L'expression « quantité thérapeutiquement efficace » signifie le taux ou la quantité de composé nécessaire et suffisante pour ralentir ou stopper la progression, l'aggravation ou la détérioration d'un ou plusieurs symptômes de la maladie ou du trouble, en particulier une maladie ou un trouble lié(e) à une baisse de la libération de calcium, en particulier une myopathie liée à une ou plusieurs mutations du gène RyRl, plus particulièrement la myopathie à cores; soulager les symptômes de la maladie ou du trouble, en particulier une myopathie liée à une ou plusieurs mutations du gène RyRl, plus particulièrement la myopathie à cores.

La « quantité thérapeutiquement efficace » dépend du sujet, du stade de la maladie à traiter et du mode d'administration, et peut être déterminée par des opérations de routine par l'homme du métier. Cette quantité peut varier avec l'âge et le sexe du sujet.

Avantageusement, une quantité thérapeutiquement efficace peut varier entre 0,01 et 100 mg/kg de masse corporelle, de préférence entre 0,1 et 20 mg/kg, et de façon davantage préférée entre 1 et 10 mg/kg, par exemple en une ou plusieurs administrations hebdomadaires, pendant un ou plusieurs mois.

En particulier, la quantité humaine thérapeutiquement efficace peut être comprise entre 50 mg/mois et 500 mg/mois pour une femme de 60 kg, de manière préférée la quantité humaine thérapeutiquement efficace est de 250 mg/mois pour une femme de 60 kg, soit de l'ordre de 4,17 mg/kg/mois.

A titre d'exemple, pour les souris, la quantité thérapeutiquement efficace a été adaptée selon la préconisation de la FDA des Etats-Unis (Food and Drug Administration), à savoir par multiplication de la quantité humaine thérapeutiquement efficace par un facteur 12,3 pour obtenir la quantité murine efficace. Ainsi, la dose murine préférée est de 51 mg/kg/mois, soit pour une souris de 20 g, une dose de 1 mg/mois. Dans le cadre de la présente invention et pour les tests réalisés sur les souris, une quantité d'environ 0,25 mg/semaine a été administrée par injection intramusculaire.

En outre, la quantité thérapeutiquement efficace spécifique pour n'importe quel patient dépendra d'une diversité de facteurs comprenant le trouble traité et la gravité du trouble ; l'activité du composé spécifique utilisé ; la composition spécifique utilisée, l'âge, la masse corporelle, l'état de santé général, le sexe et le régime du patient ; la durée d'administration, la voie d'administration, et le taux d'excrétion du composé spécifique utilisé ; la durée du traitement ; les médicaments utilisés en combinaison ou simultanément avec le composé spécifique utilisé ; et les facteurs similaires bien connus dans la technique médicale. Par exemple, il est bien dans les compétences de l'homme du métier de commencer des doses du composé à des taux inférieurs à ceux requis pour obtenir l'effet thérapeutique désiré et d'augmenter progressivement le dosage jusqu'à ce que l'effet désiré soit obtenu.

Selon un autre aspect, l'antagoniste de la présente invention est destiné à une utilisation en tant que médicament.

L'invention concerne également une composition pharmaceutique comprenant :
- au moins l'antagoniste du récepteur des oestrogènes de la présente invention ;
- au moins un excipient pharmaceutiquement acceptable.

L'expression "excipient pharmaceutiquement acceptable" se réfère à un matériel non toxique qui est compatible avec un système biologique tel qu'une cellule, une culture cellulaire, un tissu ou un organisme. Cet excipient pharmaceutiquement acceptable ne produit pas de réaction indésirable, allergique ou autre lorsqu'il est administré à un animal, en particulier un être humain. Les caractéristiques de l'excipient dépendront du mode d'administration utilisé.

Ceci comprend n'importe quel solvant, diluant, milieu de dispersion, agglutinant, liant, lubrifiant, délitant, revêtement, agent antibactérien et antifongique, agent isotonique et agent retardateur d'absorption, et adjuvants similaires. Un excipient pharmaceutiquement acceptable se réfère à une charge solide, semi-solide ou liquide non-toxique, un diluant, une matière d'encapsulation ou une formulation accessoire de tout type. Pour l'administration humaine, les préparations devront satisfaire les exigences de stérilité, pyrogénicité, de sûreté générale et de pureté telles que requises par les Bonnes pratiques de fabrication des substances actives à usage humain et vétérinaire.

Selon un aspect, la composition pharmaceutique est destinée à être administrée à un sujet selon une quantité thérapeutiquement efficace.

Dans les compositions pharmaceutiques de la présente invention, le principe actif, seul ou en combinaison avec un autre principe actif, peut être administré sous une forme d'administration unitaire, sous la forme d'un mélange avec des supports pharmaceutiques classiques, à des animaux et des êtres humains. Les formes d'administration unitaires appropriées comprennent les formes adaptées à la voie orale telles que les comprimés, les gélules, les poudres, les granules et les suspensions ou solutions pour voie orale, les formes d'administration sublinguale et buccale, les aérosols, les implants, les formes d'administration sous-cutanée, transdermique, topique, intrapéritonéale, intramusculaire, intraveineuse, sous-dermique, transdermique, intrathécale et intranasale et les formes d'administration rectale.

Selon un autre aspect, la composition pharmaceutique est destinée à une utilisation en tant que médicament.

A cet effet, la composition pharmaceutique ou le médicament contient des véhicules qui sont pharmaceutiquement acceptables pour une formulation adaptée pour l'administration par voie orale.

Des exemples de formes adaptées pour l'administration par voie orale comprennent, mais sans y être limitées, des comprimés, des comprimés à orodispersion, des comprimés effervescents, des poudres, des granules, des pilules (comprenant des pilules édulcorées), des dragées, des gélules (comprenant des gélules de gélatine molle), des sirops, des liquides, des gels ou d'autres solutions, des suspensions, des bouillies, des formes liposomales et similaires.

Dans un mode de réalisation, la composition pharmaceutique ou le médicament contient des véhicules qui sont pharmaceutiquement acceptables pour une formulation apte à être injectée.

Des exemples de formes adaptées à l'injection comprennent, mais sans y être limitées, des solutions, telles que, par exemple, des solutions aqueuses stériles, des dispersion, des émulsions, des suspensions, des formes solides appropriées pour l'utilisation pour préparer des solutions ou suspensions par l'ajout d'un liquide avant l'utilisation, par exemple, une poudre, des formes liposomales ou similaires.

Le mode d'administration peut être par injection ou par perfusion graduelle. L'injection peut être intraveineuse, intra-péritonéale, intramusculaire, sous-cutanée ou transdermale.

Les préparations pour une administration parentérale peuvent inclure des solutions aqueuses ou non-aqueuses stériles, des suspension ou des émulsions. Des exemples de solvants non-aqueux sont l'alcool benzylique, l'éthanol, le propylène glycol, le polyéthylène glycol, des huiles végétales ou des esters organiques injectables tels que l'éthyl oléate. Des véhicules aqueux comprennent l'eau, des solutions alcool/eau, des émulsions ou des suspensions.

L'invention concerne également une méthode de traitement d'une maladie ou d'un trouble lié(e) à une baisse de la libération de calcium entre le réticulum sarcoplasmique et le cytosol, en particulier une myopathie liée à une ou plusieurs mutations du gène RyRl ou une myopathie liée à une baisse de la libération de calcium, plus particulièrement la myopathie à cores.

La méthode de traitement de la présente invention comprend l'administration à un sujet en ayant besoin d'une quantité thérapeutiquement efficace de l'agoniste du récepteur des oestrogènes de formule :

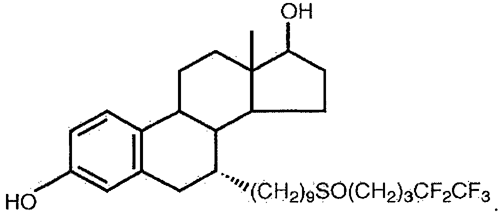

Dans un autre mode de réalisation, la méthode de traitement de la présente invention comprend l'administration à un sujet en ayant besoin d'une quantité thérapeutiquement efficace de la composition pharmaceutique de l'invention.

L'invention concerne ainsi une méthode de traitement d'un patient souffrant d'une maladie ou d'un trouble lié(e) à une baisse de la libération de calcium entre le réticulum sarcoplasmique et le cytosol, en particulier une myopathie liée à une ou plusieurs mutations du gène RyRl ou une myopathie liée à une baisse de la libération de calcium, plus particulièrement la myopathie à cores, comprenant l'administration d'une quantité thérapeutiquement efficace de l'agoniste du récepteur des oestrogènes de l'invention ou de la composition pharmaceutique de l'invention.

L'administration de l'agoniste du récepteur des oestrogènes ou de la composition pharmaceutique de l'invention peut se faire par toute voie d'administration mentionnée plus haut.

Pour mieux illustrer l'objet de la présente invention, on va maintenant en décrire ci-dessous, à titre illustratif et non limitatif, les exemples ci-après en liaison avec les dessins annexés.

Sur ces dessins :
La Figure 1 est un schéma de la construction du modèle de souris KO inductible RyRl.
La Figure 2 est une représentation graphique des mesures de fluorescence en imagerie calcique sur cellules murines.
La Figure 3A est une représentation graphique des mesures de fluorescence en imagerie calcique sur cellules murines, en présence ou en absence de traitement avec le fulvestrant 100 nM.
La Figure 3B est un graphique à barres représentant l'amplitude du pic de fluorescence en imagerie calcique, en présence ou en absence de traitement avec le fulvestrant 100 nM.
La Figure 4A est une représentation graphique des mesures de fluorescence en imagerie calcique sur cellules humaines témoins CTRL, en présence ou en absence de traitement avec le fulvestrant 100 nM.
La Figure 4B est une représentation graphique des mesures de fluorescence en imagerie calcique sur cellules humaines témoins CTRL, en présence ou en absence de traitement avec le fulvestrant 250 nM.
La Figure 4C est une représentation graphique des mesures de fluorescence en imagerie calcique sur cellules humaines immortalisées MELA issues d'une biopsie d'un patient atteint de myopathie à cores, en présence ou en absence de traitement avec le fulvestrant 100 nM.
La Figure 4D est une représentation graphique des mesures de fluorescence en imagerie calcique sur cellules humaines immortalisées MELA issues d'une biopsie d'un patient atteint de myopathie à cores, en présence ou en absence de traitement avec le fulvestrant 250 nM.
La Figure 4E est un graphique à barres représentant l'amplitude du pic de fluorescence en imagerie calcique sur cellules humaines témoins CTRL, en présence ou en absence de traitement avec le fulvestrant 100 nM ou 250 nM.
La Figure 4F est un graphique à barres représentant l'amplitude du pic de fluorescence en imagerie calcique sur cellules humaines MELA, en présence ou en absence de traitement avec le fulvestrant 100 nM ou 250 nM.
La Figure 5 est un graphique représentant la force des souris post-induction de la myopathie à cores mesurée en durée d'agrippement au cours du temps, en présence ou en absence de traitement avec le fulvestrant 100 nM.

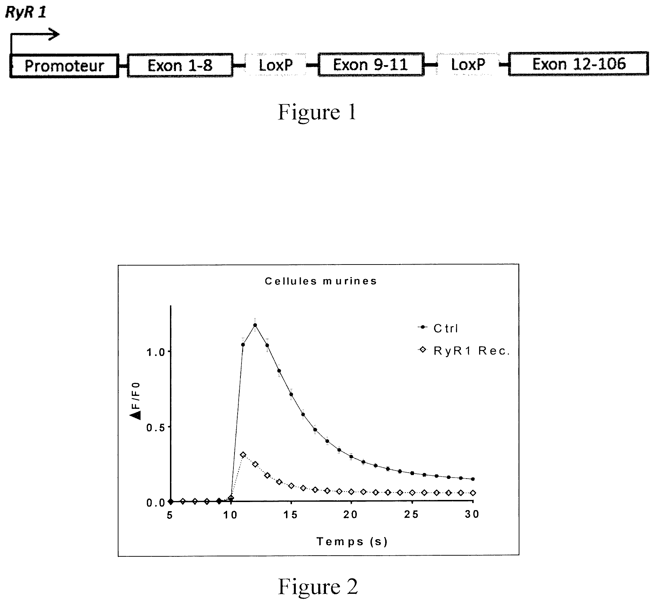

Sur la Figure 1, on peut voir la schématisation de la construction génétique du modèle de souris KO inductible RyRl développé par les inventeurs. Les exons 9-11 du gène RYR1 sont flanqués de sites LoxP, sur les deux allèles du gène. Les souris possèdent également un transgène HSA-Cre/ERT2 codant pour la Cre recombinase. L'inactivation du gène par KO est induite par des injections intrapéritonéales de tamoxifène. Ainsi, en présence de tamoxifène, la Cre recombinase est transloquée vers le noyau et le système de recombinaison Cre-Lox permet de réaliser une délétion des exons 9-11 du gène RYR1. Ce modèle de souris KO inductible développé au laboratoire est non létal et constitue un modèle d'étude de la myopathie à cores.

Si l'on se réfère à la Figure 2, en imagerie calcique, les cellules sont chargées pendant 30 minutes avec la sonde Fluo-4 qui, en fixant les ions Ca²⁺, émet une fluorescence proportionnelle à la concentration cytoplasmique de calcium. Les relâchements calciques du réticulum sarcoplasmique vers le cytosol sont donc suivis en direct sur des cellules vivantes par vidéo-microscopie après une stimulation par du KCl à 140 mM (mime une dépolarisation membranaire). On visualise ainsi, sur des cellules murines en culture primaire, que l'induction de l'inactivation du KO du gène RYR1 (cellules RyRl recombinées) entraîne une réduction significative du pic de relâchement de calcium, réduction qui a été chiffrée à 73% par rappport au relâchement de calcium dans les cellules murines témoins (Ctrl).

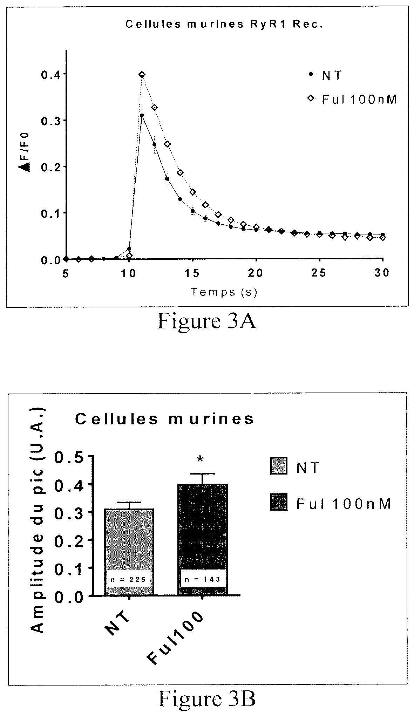

D'une manière similaire, des études complémentaires en imagerie calcique ont été conduites sur ces cellules murines en culture primaire, après induction de la recombinaison par transduction par AdV-Cre (cellules RyRl recombinantes). Ainsi, on peut observer sur la Figure 3A que le fulvestrant à 100 nM améliore le relâchement calcique par rapport aux cellules non traitées (NT). La Figure 3B montre l'amplitude du pic de relâchement calcique, lequel passe de 0,31 +/- 0,02 à 0,40 +/- 0,04, soit une augmentation de 28% (Test de Student, p < 0,05). Le nombre de myotubes analysés dans chaque condition est indiqué dans les barres du graphique.

Les mêmes expériences ont été réalisées sur des cellules musculaires humaines témoins CTRL et sur des cellules MELA, qui sont des cellules humaines immortalisées issues d'une biopsie de patient atteint de myopathie à cores.

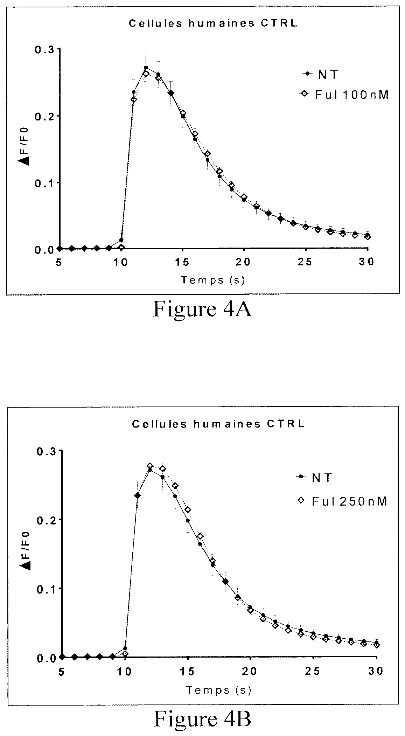

Si l'on se réfère à la Figure 4A, on peut y voir que le traitement par le fulvestrant 100 nM n'a pas d'effet sur les cellules témoins. De manière similaire, le traitement par le fulvestrant 250 mM n'a pas non plus d'effet sur les cellules témoins (Figure 4B).

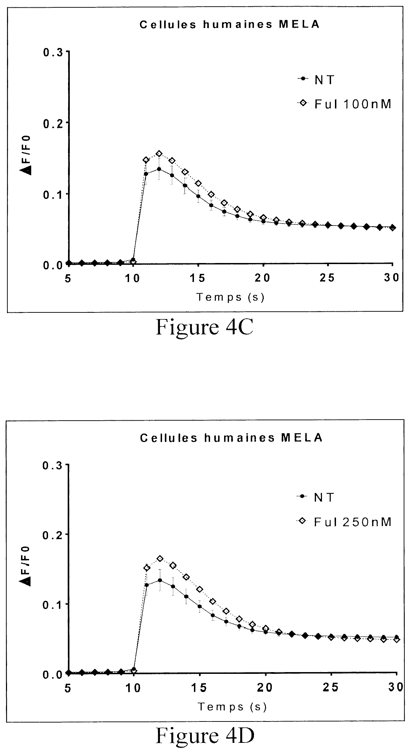

En revanche, on peut visualiser sur la Figure 4C que le pic de relâchement de calcium des cellules humaines MELA est réduit de manière importante par rapport aux cellules témoins, que les cellules soient traitées ou non par le fulvestrant 100 nM. On peut noter que le traitement par le fulvestrant 100 nM tend à augmenter le relâchement de calcium, mais de manière non statistiquement significative. La même observation peut être faite de la Figure 4D : le relâchement de calcium s'est effondré dans les cellules humaines MELA et le traitement avec le fulvestrant 250 nM tend également à augmenter le relâchement de calcium par rapport aux cellules non traitées, mais les données statistiques ne se sont pas révélées significatives.

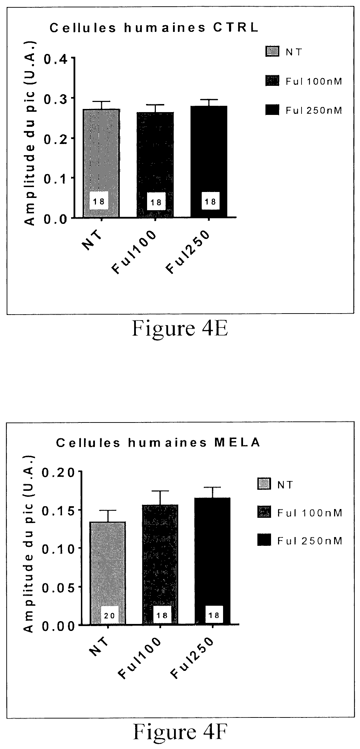

Si l'on se réfère à la Figure 4E, le graphique montre que le traitement par le fulvestrant, que ce soit à une concentration de 100 nM ou 250 nM, n'a pas d'effet sur les cellules musculaires humaines témoins. Le nombre de puits analysés dans chaque condition est indiqué dans les barres du graphique.

En revanche, si l'on se réfère à la Figure 4F, le graphique permet de visualiser la tendance à l'amélioration du relâchement de calcium des cellules humaines MELA en réponse à un traitement par le fulvestrant 100 nM et plus encore par le fulvestrant 250 nM. On note également que le pic de relâchement de calcium est passé d'environ 0,28 chez des cellules musculaires humaines témoins à environ 0,15 chez les cellules immortalisées issues d'une biopsie de patient atteint de myopathie à cores.

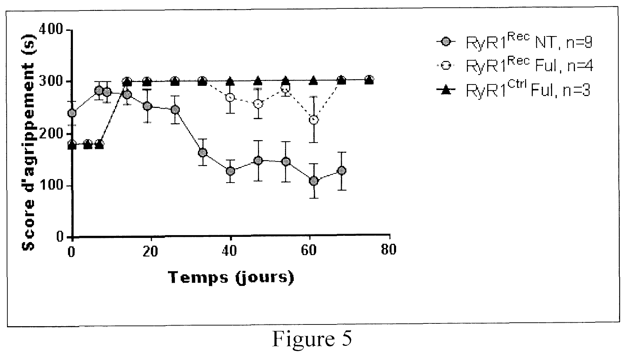

Pour finir, si l'on se réfère à la Figure 5, on peut y voir que la durée d'agrippement des souris chez qui on a induit la myopathie à cores (RyR1^{Rec}) traitées avec le fulvestrant 100 nM est améliorée par rapport aux souris non traitées (NT). La force des souris traitées au fulvestrant est nettement améliorée par rapport aux souris non traitées.

### EXEMPLES

Les exemples suivants illustrent l'invention.

### Matériels et Méthodes

### Modèle animal

Pour cette étude, un modèle inductible et non-létal de souris KO pour RyRl développé au laboratoire a été utilisé. Chez ces souris, les exons 9 à 11 du gène RYR1 sont flanqués de sites LoxP sur les deux allèles (RYR1 fl/fl). Ces souris possèdent également un transgène HSA-Cre/ERT2 (RyR1^{Rec}) codant pour la Cre recombinase, alors que cette construction est absente chez les souris témoins (RyR1^{ctrl}) . Le KO a été induit par des injections intrapéritonéales de tamoxifène (Sigma) huit semaines après la naissance, à raison de 1 mg/jour pendant cinq jours. Il est important de noter que tous les animaux ont reçu ces injections, qu'ils possèdent ou non l'allèle HSA-Cre.

De façon exclusive chez les animaux possédant l'allèle HSA-Cre, le tamoxifène (TAM) provoque la translocation de la Cre recombinase vers le noyau, permettant ainsi la recombinaison des sites LoxP et la délétion des exons 9-11 du gène RYR1 (Figure 1). Afin d'améliorer la compréhension, les souris chez lesquelles la recombinaison ne peut pas s'opérer seront dénommées souris RyR1^{Ctrl} dans la suite du document. Les souris recombinées après l'administration intrapéritonéale de TAM seront dénommées souris RyR1^{Rec}.

### Traitements des animaux

Pour le traitement des animaux, le fulvestrant a été préparé à partir d'une solution commerciale de Faslodex 250 mg (AstraZeneca). Cette solution a été diluée dans du sérum physiologique et 0,27 mg de fulvestrant a été injecté à chaque souris (poids de 20 g) dans 50 µl de volume. Les injections ont été réalisées par voie intramusculaire dans le quadriceps une fois par semaine.

Le choix de la dose injectée a été fait conformément à la préconisation de la FDA indiquant d'utiliser une dose 12,3 fois supérieure à celle de la dose recommandée chez l'homme. Ici, pour une dose humaine de 250 mg/mois chez une femme de 60 kg, la dose rapportée au poids chez la souris est de 51 mg/kg/mois, soit 1 mg/mois pour une souris de 20 g, réparti en 4 injections, par conséquent environ 0,25 mg/semaine.

### Test d'agrippement sur les animaux

Afin d'évaluer la force musculaire des animaux, les souris ont été placées sur une grille qui a ensuite été retournée. Le durée pendant laquelle les souris restent agrippées à la grille a été chronométrée dans une limite de 300 secondes. Cette mesure a été réalisée de façon hebdomadaire sur l'ensemble des souris de l'étude.

### Cultures cellulaires

### Cellules satellites murines primaires

Les cellules satellites ont été isolées à partir des muscles squelettiques des membres inférieurs de souris nouveau-nées RyR1^{Ctrl} selon la procédure décrite dans Marty et al., 2000.

Les cellules ont été ensemencées dans des plaques 96 puits (75 000 cellules/puits) préalablement recouvertes de laminine. Les cellules ont d'abord été cultivées dans du milieu de prolifération composé de *Ham's F-10 Nutrient Mix* (Life Technologies), 20% de sérum de veau foetal (Life technologies), 2% d'Ultroser G (PALL) et 2% de Pénicilline-Streptomycine (Life Technologies).

Les cellules RyR1^{Ctrl} ainsi produites n'expriment pas la Cre recombinase. Pour induire la recombinaison du gène RyRl floxé, les cellules ont donc été transduites 12 heures après ensemencement avec des adénovirus permettant l'expression de la Cre recombinase, AdV-Cre (Utah University), à une multiplicité d'infection (*multiplicity of infection,* ou MOI) de 64.

24 heures après l'ensemencement, les cellules ont été placées dans du milieu de différenciation composé de *Dulbecco's Modified Eagle Medium* (Life Technologies), 2% de sérum de cheval (Life Technologies) et 1% de Pénicilline-Streptomycine (Life Technologies) pendant 3 jours.

### Cellules humaines

Les cellules CTRL sont des cellules humaines immortalisées issues d'une biopsie d'un sujet sain ne présentant pas d'anomalie de relâchement calcique.

Les cellules MELA sont des cellules humaines immortalisées issues d'une biopsie d'un patient atteint de myopathie à cores, présentant notamment un défaut quantitatif de RyRl ainsi qu'un défaut de relâchement calcique (Rendu et al., 2013). Ces cellules ont été immortalisées par l'équipe de Vincent Mouly (Institut de Myologie, Paris).

Les cellules ont été ensemencées dans des plaques 96 puits (50 000 cellules/puits) dans du milieu de prolifération de composition identique à celui des cellules murines primaires.

24 heures après l'ensemencement, les cellules ont été placées dans du milieu de différenciation pendant 7 jours. Le milieu a été renouvelé 4 jours après la mise en différenciation.

### Traitement des cultures

Le fulvestrant (Tocris) a été solubilisé dans du diméthylsulfoxide à une concentration de 10 mg/ml. Cette solution stock a été conservée à -20°C et utilisée pour les tests cellulaires après dilution dans le milieu de différenciation des cellules à la concentration souhaitée. Les cellules ont été traitées pendant toute la durée de la différenciation.

### Imagerie calcique

Les cellules ont été chargées pendant 30 minutes avec du Fluo-4 Direct (Thermofisher). Les relâchements calciques sont suivis avec un vidéo-microscope Leica DMI6000 FRAP (Leica Microsystems) pendant une durée de 40 secondes, avec une stimulation par du KCl à 140 mM.

Les films ont été analysés grâce aux logiciels Fiji (Schindelin et al., 2012) et Prism (GraphPad).

Pour les cellules murines, les mesures de fluorescence ont été réalisées sur des régions d'intérêt correspondant chacune à un myotube, avec plusieurs dizaines de myotubes mesurés pour chaque répétition de l'expérience.

Pour les cellules humaines, la forme des cellules ne permettant pas de les distinguer individuellement de manière précise, les mesures ont été réalisées sur une seule région d'intérêt par puits correspondant à la surface totale couverte par les myotubes. 4 à 6 puits ont été mesurés pour chaque répétition de l'expérience.

### Analyses statistiques

Les comparaisons ont été réalisées avec le test de Student en utilisant le logiciel Prism (GraphPad). Les différences sont considérées comme significatives lorsque p<0,05.

### Exemple 1 : Etude de la libération (ou relâchement) calcique in vitro sur cellules murines primaires

Les cellules RyR^{Ctrl} transduites ou non par la Cre recombinase AdV-Cre ont été traitées avec les différents agents, depuis la mise en différenciation jusqu'au moment de l'analyse fonctionnelle. L'intensité du relâchement calcique a été évaluée en réponse à une stimulation par le KCl 140 mM (mimant une dépolarisation à ∼0mV).

Comme attendu, l'expression de la Cre-recombinase s'accompagne de la baisse des capacités de relâchement calcique des myotubes. En effet, comme le montre la Figure 2, l'induction de l'inactivation par KO du gène RyRl s'accompagne d'une réduction de 73% de l'amplitude du pic de relâchement de calcium (p < 0,01).

Les cellules murines RyR^{CTRL} après induction de la recombinaison par transduction par AdV-Cre (cellules RyRl-Rec) sont placées en milieu de différenciation en absence (NT) ou en présence de fulvestrant 100 nM (Ful 100nM). Après 3 jours de différenciation, les relâchements de calcium ont été étudiés en imagerie calcique (Figure 3A). Le fulvestrant à 100 nM améliore le relâchement calcique, le pic passe de 0,31 +/- 0,02 à 0,40 +/- 0,04, soit une augmentation de 28% (p < 0,05) (Figure 3B).

### Exemple 2 : Etude de la libération (ou relâchement) calcique in vitro sur cellules humaines

Le fulvestrant a été testé sur des cellules musculaires humaines présentant ou non un défaut de relâchement calcique (cellules humaines CTRL ou MELA), par une incubation de 7 jours en milieu de différenciation.

Comme le montrent les Figures 4A et 4B, le fulvestrant n'a pas d'effet sur les cellules témoins CTRL. Dans les cellules MELA, dont les relâchements de calcium sont effondrés, il tend à augmenter les relâchements de calcium aussi bien à une concentration de 100 nM qu'à 250 nM (Figures 4C-4E). Cependant, la quantification étant faite non plus sur chaque myotube mais sur chaque puits de la plaque de culture, le nombre d'échantillons est plus faible et la différence n'atteint pas la significativité statistique.

### Exemple 3 : Mesure de la force in vivo chez les souris traitées avec le fulvestrant

Les souris femelles (souris RyR1^{CTRL} ou souris RyR1 ^{Rec}) ont été traitées soit par le fulvestrant 100 nM (Ful) soit par du sérum physiologique (NT) en injection intramusculaire une fois par semaine pendant toute la durée de l'expérience.

La force des animaux est suivie par un grip test (test d'agrippement) une fois par semaine, juste avant les injections. Les résultats de la mesure de force au cours du temps sont présentés sur la Figure 5.

Les résultats obtenus sur ce petit groupe de souris femelles traitées au fulvestrant montrent une amélioration très nette de la force des souris par rapport aux souris non traitées. Les analyses statistiques pourront être réalisées sur un nombre d'animaux plus important, englobant également des individus mâles.

### Références bibliographiques:

Berry M, Metzger D, Chambon P, Role of the two activating domains of the oestrogen receptor in the cell-type and promoter-context dépendent agonistic activity of the antioestrogen 4-hydroxytamoxifen, EMBO J. 1990 Sep; 9(9): 2811-2818.
De Luca A, Pierno S, Liantonio A, Cetrone M, Camerino C, Simonetti S, Papadia F, Camerino DC, Alteration of excitation-contraction coupling mechanism in extensor digitorum longus muscle fibres of dystrophic mdx mouse and potential efficacy of taurine, Br J Pharmacol. 2001 Mar;132(5):1047-54.
Dorchies OM, Reutenauer-Patte J, Dahmane E, Ismail HM, Petermann O, Patthey- Vuadens O, Comyn SA, Gayi E, Piacenza T, Handa RJ, Décosterd LA, Ruegg UT, The anticancer drug tamoxifen counteracts the pathology in a mouse model of duchenne muscular dystrophy, Am J Pathol. 2013 Feb;182(2):485-504.
Dowling JJ, Low SE, Busta AS, Feldman EL, Zebrafish MTMR14 is required for excitation-contraction coupling, developmental motor function and the regulation of autophagy, Hum Mol Genet. 2010 Jul 1;19(13):2668-81.
Dowling JJ, Vreede AP, Low SE, Gibbs EM, Kuwada JY, Bonnemann CG, Feldman EL, Loss of myotubularin function results in T-tubule disorganization in zebrafish and human myotubular myopathy, PLoS Genet. 2009 Feb;5(2):e1000372.
Kulkoyluoglu E, Madak-Erdogan Z, Nuclear and extranuclear-initiated estrogen receptor signaling crosstalk and endocrine résistance in breast cancer, Steroids. 2016 Oct;114:41-47.
Plant DR, Lynch GS, Depolarization-induced contraction and SR function in mechanically skinned muscle fibers from dystrophic mdx mice, Am J Physiol Cell Physiol. 2003 Sep;285(3):C522-8.
Rendu J, Brocard J, Denarier E, Monnier N, Piétri-Rouxel F, Beley C, Roux-Buisson N, Gilbert-Dussardier B, Perez MJ, Romero N, Garcia L, Lunardi J, Fauré J, Fourest-Lieuvin A, Marty I, Exon skipping as a therapeutic strategy applied to an RYR1 mutation with pseudo-exon inclusion causing a severe core myopathy, Hum Gene Ther. 2013 Jul;24(7):702-13. Salvatori L, Pallante P, Ravenna L, Chinzari P, Frati L, Russo MA, Petrangeli E, Oestrogens and selective oestrogen receptor (ER) modulators regulate EGF receptor gene expression through human ER alpha and beta subtypes via an Sp1 site, Oncogene, 2003 Jul 31;22(31):4875-81.
Schindelin, J.; Arganda-Carreras, I. & Frise, E. et al. (2012), Fiji: an open-source platform for biological-image analysis, Nature methods 9(7): 676-682.

## Revendications

1. Antagoniste du récepteur des oestrogènes de formule : 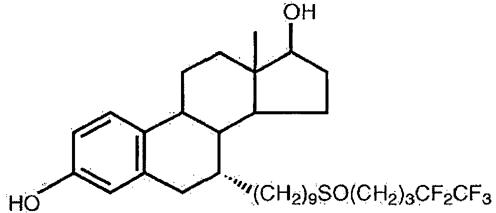 pour l'utilisation dans le traitement d'une maladie ou d'un trouble lié(e) à une baisse de la libération de calcium entre le réticulum sarcoplasmique et le cytosol.

2. Antagoniste du récepteur des oestrogènes selon la revendication 1, dans lequel la maladie ou le trouble est une myopathie liée à une ou plusieurs mutations du gène RyRl ou une myopathie liée à une baisse de la libération de calcium.

3. Antagoniste du récepteur des oestrogènes selon l'une des revendiations 1 ou 2, dans lequel la maladie ou le trouble est la myopathie à cores.

4. Antagoniste du récepteur des oestrogènes, selon l'une quelconque des revendications 1 à 3, **caractérisé par le fait que** l'antagoniste est destiné à être administré à un sujet selon une quantité thérapeutiquement efficace.

5. Antagoniste du récepteur des oestrogènes, selon l'une quelconque des revendications 1 à 4, **caractérisé par le fait que** l'antagoniste est destiné à une utilisation en tant que médicament.

6. Composition pharmaceutique comprenant :
- au moins l'antagoniste du récepteur des oestrogènes selon l'une quelconque des revendications 1 à 3 ;
- au moins un excipient pharmaceutiquement acceptable.

7. Composition pharmaceutique selon la revendication 6, **caractérisée par le fait que** la composition pharmaceutique est destinée à être administrée à un sujet selon une quantité thérapeutiquement efficace.

8. Composition pharmaceutique selon l'une quelconque des revendications 6 ou 7, **caractérisée par le fait que** la composition pharmaceutique est destinée à une utilisation en tant que médicament.
